# EUROPEAN PATENT SPECIFICATION

(11) **EP 3 280 449 B1**
(45) Date of publication and mention of the grant of the patent: **20.11.2019**
(21) Application number: 16777049.4
(22) Date of filing: 29.03.2016
(51) Int. Cl.: A61K 47/10, A61K 47/14, A61K 9/10, A01N 31/04, A01N 25/04, A01N 25/30, A61K 8/34, A61K 8/39, A61Q 13/00, A61Q 15/00, A61Q 17/00, A61Q 19/00, A61K 8/02, C11B 9/00, C11D 1/83, C11D 3/20, C11D 3/48, C11D 3/50, C11D 17/00

(54) **AN AQUEOUS MULTILAMELLAR COMPOSITION FOR DELIVERING HYDROPHOBIC SUBSTANCES**
WÄSSRIGE MULTILAMELLARE ZUSAMMENSETZUNG ZUR VERABREICHUNG VON HYDROPHOBEN SUBSTANZEN
COMPOSITION MULTILAMELLAIRE AQUEUSE DESTINÉE À ADMINISTRER DES SUBSTANCES HYDROPHOBES

(30) Priority: 09.04.2015 US 201562145340 P
(43) Date of publication of application: 14.02.2018
(73) Proprietor: ISP Investments LLC, Wilmington, DE 19805 (US)
(72) Inventor: KONATE, Nadia, 39108 Magdeburg (DE); PREMACHANDRAN, Raman, White Plains, New York 10605 (US); WINKOWSKI, Karen, Springfield, New Jersey 07081 (US); WINGENFELD, Andrea, 87493 Lauben (DE)
(74) Representative: Kutzenberger Wolff & Partner
(86) International application number: PCT/US2016/024704
(87) International publication number: WO 2016/164205

(56) References cited:
- EP-A1- 2 774 481
- WO-A1-2014/025413
- WO-A2-2013/105047
- US-A- 5 853 755
- US-A1- 2012 328 541
- US-A1- 2013 224 125
- US-A1- 2014 335 137

## Description

### FIELD OF THE INVENTION

The present application relates to a multilamellar composition, and more particularly, to an aqueous multilamellar composition for delivering hydrophobic substances such as phenylethylalcohol and/or phenylpropylalcohol.

### BACKGROUND OF THE INVENTION

Phenyl ethyl alcohol and phenyl propyl alcohol are typical fragrance components of personal care, household, industrial & institutional products and the like. These substances have exhibited preservatives and/or antimicrobial boosting properties. However, use of phenyl ethyl alcohol and phenyl propyl alcohol substances limit the formulators with difficulties such as poor solubility in water, loss of activity on evaporation and partitioning to the packaging material and strong odor due to their high vapor pressure.

In view of the above, there remains a need to find a solution to increase the solubility, prevent the loss of activity and reduce the vapor pressure of phenyl ethyl alcohol and phenyl propyl alcohol.

Accordingly, it is a primary objective of the present application to provide a solution to enhance the water solubility of poorly soluble phenyl ethyl alcohol and phenyl propyl alcohol fragrance substances.

Another objective of the present application is to reduce the vapor pressure of phenyl ethyl alcohol and phenyl propyl alcohol compounds.

Yet another objective of the present application is to prevent the loss of activity of phenyl ethyl alcohol and phenyl propyl alcohol compounds.

Still another objective of the present application is to employ a completely natural and eco certified surfactant system for preparing multilamellar vesicle composition.

WO2013105047A2 discloses use of zerumbone for a non therapeutical cosmetic topical treatment of skin firming via the protection and repairing of the extracellular matrix (ECM) of the dermis, and wherein, the treatment for the topical application employs zemea (Propanediol) and NatraGem S140 (Polyglyceryl-4 Laurate/Sebacate (and) Polyglyceryl-6 Caprylate/Caprate (and) Aqua.

WO2012055855 A1 discloses concentrated preparations of 1-60% by weight of the Ethyl Lauroyl Arginate HCI (LAE) comprising (i), 1 to 99 % by weight of at least one solvent or surfactant selected from the group consisting of phenoxyethanol, benzyl alcohol, caprylyl glycol, hexylene glycol, pentylene glycol, decylene glycol, glyceryl monoester with a fatty acid selected from caprylic acid, capric acid, undecylenic acid and lauric acid, polyglycerine monoesters (polyglyceryl-3 caprylate), phenethyl alcohol, phenyl propanol and ethylhexyl glycerine, provided that when phenoxyethanol is present as the at least one solvent or surfactant its concentration shall be 2 to 99% by weight; (ii) optionally 0-20 % by weight of one or more organic acids with antibacterial activity; (iii) optionally 0-10 % by weight of one or more natural extracts with antibacterial activity; (iv) optionally 0-10 % by weight of one or more chelating agents; (v) optionally 0-10 % by weight of one or more antioxidants; and (vi) optionally 0-50% by weight of one or more solvents or surfactants without antibacterial activity. These concentrates may be added to cosmetic compositions for personal care as preserving agents.

### SUMMARY OF THE INVENTION

The primary objective of the present application is to provide an effective delivery system for phenylethylalcohol and/or phenylpropyl alcohol, typical fragrance components of many end-user applications relating to personal care, house hold, cleaning, and industrial and institutional products.

Another objective of the present application is to provide an aqueous multilamellar composition for delivering a hydrophobic substance comprising: (i) about 50 *wt.* % to about 80 *wt.* % of phenylethylalcohol and/or phenylpropylalcohol; (ii) a mixture of (a) about 10 *wt.* % to about 20 *wt.* % of polyglyceryl-4 laurate/sebacate and (b) about 10 *wt.* % to about 20 *wt.* % of polyglyceryl-6 caprylate/caprate; (iii) about 10 *wt.* % to about 20 *wt.* % of octane-1,2-diol; (iv) optionally about 10 *wt.* % to about 20 *wt.* % of 1,3-propanediol; and (v) about 5.0 *wt.* % to about 80 *wt.* % of water.

One other aspect of the present application is to provide a multilamellar composition comprising phenylethylalcohol and/or phenylpropylalcohol which is capable of inhibiting or killing yeasts, mold spores, *gram* (+), *gram* (-) bacterial strains, acne causing strains, or odor causing strains.

Yet another aspect of the present application provides a process for preparing an aqueous multilamellar composition, wherein said process comprises mixing: (i) about 50 *wt.* % to about 80 *wt.* % of phenylethylalcohol and/or phenylpropylalcohol; (ii) a mixture of (a) about 10 *wt.* % to about 20 *wt.* % of polyglyceryl-4 laurate/sebacate and (b) about 10 *wt.* % to about 20 *wt.* % of polyglyceryl-6 caprylate/caprate; (iii) about 10 *wt.* % to about 20 *wt.* % of octane-1,2-diol; (iv) optionally about 10 *wt.* % to about 20 *wt.* % of 1,3-propanediol; and (v) about 5 *wt.* % to about 80 *wt.* % of water.

Still another aspect of the present application relates to a method of killing bacteria, fungi, molds, yeasts and viruses or inhibiting their growth in a cosmetic, personal care, house hold, cleaning, and/or Industrial & Institutional products those are susceptible to growth of microorganisms.

One important aspect of the present application provides a method for controlled or periodic delivery of a hydrophobic substance of the present application if they are incorporated into cosmetic or personal care or house hold, cleaning, Industrial & Institutional products.

### DETAILED DESCRIPTION OF THE INVENTION

While this specification concludes with claims particularly pointing out and distinctly claiming that which is regarded as the invention, it is anticipated that the invention can be more readily understood through reading the following detailed description of the invention and study of the included examples.

By the term "comprising" herein is meant that various optional, compatible components can be used in the compositions herein, provided that the important ingredients are present in the suitable form and concentrations. The term "comprising" thus encompasses and includes the more restrictive terms "consisting of" and "consisting essentially of" which can be used to characterize the essential ingredients such as phenylethylalcohol, phenylpropylalcohol, polyglyceryl-4 laurate/sebacate, polyglyceryl-6 caprylate/caprate, octane-1,2-diol, and 1,3-propanediol of the multilamellar composition.

All percentages, parts, proportions and ratios as used herein, are by weight of the total composition, unless otherwise specified. All such weights as they pertain to listed ingredients are based on the active level and, therefore; do not include solvents or byproducts that may be included in commercially available materials, unless otherwise specified.

All references to singular characteristics or limitations of the present invention shall include the corresponding plural characteristic or limitation, and *vice-versa,* unless otherwise specified or clearly implied to the contrary by the context in which the reference is made.

Numerical ranges as used herein are intended to include every number and subset of numbers contained within that range, whether specifically disclosed or not. Further, these numerical ranges should be construed as providing support for a claim directed to any number or subset of numbers in that range.

As used herein, the words "preferred," "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

References herein to "one embodiment," "one aspect" or "one version" or "one objective" of the invention include one or more such embodiment, aspect, version or objective, unless the context clearly dictates otherwise.

All publications, articles, papers, patents, patent publications, and other references cited herein are hereby incorporated herein in their entireties for all purposes to the extent consistent with the disclosure herein.

The term "preservative" or "antimicrobial" as used herein is to be understood to refer to agents such as bactericides, fungicides, algicides, aquaticides, herbicide, insecticide, pesticide, plant growth regulators and the like, which are used for their ability to inhibit growth of and/or kill biological and/or microbiological species such as bacteria, fungi, algae, caterpillar, insects, larvae, mildew, rodents, spider, worm and the like.

The term 'Preservation" refers to prevent or retard any consumer product deterioration due to microbial attack. A preservative is an active ingredient that hinders or kills the growth of bacterial and fungal strains that can be present in any consumer products, and chiefly water based consumer products. Therefore, the preservative action of any consumer product is performed by employing a single preservative or mixture of preservatives to have broad spectrum antimicrobial activity.

The term "Broad Spectrum" as described herein and claims mean that the preservative compositions of the present application have ability to inhibit or kill wide range of microbial organisms which are responsible to decay or spoil any consumer products that are prone to microbial attack.

As used, herein, "stable" and "stability" mean a composition which is significantly unaffected in chemical nature, physical homogeneity and/or color upon exposure to conditions reasonably expected to be incurred in transport, storage and their use in end-user applications. Stability may be determined either by empirical observation or by suitable methods of chemical and/or physical examination that would be known to one skilled in the art.

What is described herein is an aqueous multilamellar composition for delivering a hydrophobic substance comprising: (i) about 50 *wt.* % to about 80 *wt.* % of phenylethylalcohol and/or phenylpropylalcohol; (ii) a mixture of (a) about 10 *wt.* % to about 20 *wt.* % of polyglyceryl-4 laurate/sebacate and (b) about 10 *wt.* % to about 20 *wt.* % of polyglyceryl-6 caprylate/caprate; (iii) about 10 *wt.* % to about 20 *wt.* % of octane-1,2-diol; (iv) optionally about 10 *wt.* % to about 20 *wt.* % of 1,3-propanediol; and (v) about 5.0 *wt.* % to about 80 *wt.* % of water.

The phenylethylalcohol and phenylpropylalcohol, poorly water soluble/miscible hydrophobic substances of the present application are widely used fragrance substances in many personal care, cleaning, household, industrial and institutional products, wherein said hydrophobic substances have the ability to enhance or boost the preservative/antimicrobial activity.

According to one important embodiment of the present application, it is contemplated to employ at least one phenyl alkyl alcohol compound that can act as a perfume or fragrance substance engaged as a suitable hydrophobic substance, wherein said alkyl group has a carbon chain length of from about C₁ to C₂₀.

The amount of phenylalkylalcohol or phenylethylalcohol or phenylpropylalcohol employed in the present application is in the range of from about 50 *wt.* % to about 55 *wt.* %, about 56 *wt*. % to about 60 *wt.* %, about 61 *wt.* % to about 65 *wt.* %, about 66 *wt.* % to about 70 *wt.* %, about 71 *wt.* % to about 75 *wt.* %, about 76 *wt.* % to about 80 *wt.* %.

According to another important embodiment of the present application, the hydrophobic substance can be delivered in the form of a multilamellar structure to release the hydrophobic substance in a controlled or periodic release manner. Further, the multilamellar structure is capable of increasing the water solubility of engaged hydrophobic substances by increasing their partitioning towards oil/water interface with the largest portion into the water phase. Further, the multilamellar structure eases its incorporation into end-products formulations and facilitates the delivery of the actives at the oil water interface, which in turn enhances their efficacy and improves the stability of the actives such as phenyl ethyl alcohol and phenyl propyl alcohol. Controlled and targeted delivery at the interface helps kill organisms at a faster rate with better efficacy

Further, the lamellar vesicular structure keeps the fragrance component bloom slowly reduces the high intense fragrances and delivers the actives in a controlled manner to deliver at the oil/water interface. The delivery of the active to the oil/water interface is controlled by optimizing the vesicular lamellar structure.

The multilamellar composition comprising phenyl ethyl alcohol and/or phenyl propyl alcohol is capable of reducing the evaporation of phenyl ethyl alcohol and phenyl propyl alcohol compounds, and thereby reduced loss of activity. Accordingly, the multilamellar composition can also reduce the vapor pressure of the phenyl ethyl alcohol and phenyl propyl alcohol and in turn reduced the strong or pungent odor.

In one embodiment of the present application, the multilamellar structure can be in the form of multilamellar vesicles or multilamellar liposomes, or multilamellar niosomes. Such multilamellar structure can provide advantages like stability, entrapment, efficacies and better biological activities for delivering actives.

Further it is contemplated to employ other possible delivery systems such as unilamellar vesicles/liposomes/niosomes, micro-particulates, nano-particulates, microparticles, nano-particles, microemulsion, nanoemulsion, nanospheres, nanocapsules, solid-lipid nano particles (SLN), nanostructured lipid carriers, encapsulation methods, large vesicles, micelles, reverse micelles or lamellar liquid crystalline structures.

In preferred embodiments, the multilamellar composition of the present application does not impart any significant color change to end-user products if incorporated into it.

According to another embodiment of the present application, a mixture comprising at least two non-ionic surfactants are selected to form a multilamellar vesicle system of the present application. A preferred embodiment includes a mixture of two non-ionic surfactants, wherein the first non-ionic surfactant has a HLB value of from about 8 to 12 and the second non-ionic surfactant has a HLB value of from about 14 to 17. Another relevant embodiment employs a first non-ionic surfactant with a HLB value of from about 10 to 12 and a second non-ionic surfactant with a HLB value of from about 14 to 16. Further, the ratio of first to second non-ionic surfactant is from about 1:10 to about 10:1.

An important embodiment employs a mixture of (a) about 10 *wt.* % to about 20 *wt.* % of polyglyceryl-4 laurate/sebacate, a first non-ionic surfactant and (b) about 10 *wt.* % to about 20 *wt.* % of polyglyceryl-6 caprylate/caprate, a second non-ionic surfactant. The surfactant of the present application is commercially available as Natragem S140 NP from Croda Personal Care. It is 100% naturally derived and approved natural by Ecocert. Other non-limiting commercial products that can be employed include NatraGemTM E145 and NatraGemTM S150. Moreover, NatraGem S140 is widely accepted by Bra Miljöval (Good environmental choice), Nordic Ecolabelling (Swan) and EU Ecoflower (2014/893/EU).

Further, it is contemplated to employ other polyglycerol fatty acid esters as a non-ionic surfactants for the present application, including, but not limited to, polyglycerol esters of fatty acids, for example C3 to C22 fatty acids, such as Propionicacid, Butyricacid, Valericacid, Caproicacid, Enanthicacid, Caprylicacid, Pelargonicacid, Capricacid, Undecylicacid, Lauricacid, Tridecylicacid, Myristicacid, Pentadecanoicacid, Palmiticacid, Margaricacid, Stearicacid, Nonadecylicacid, Arachidicacid, Heneicosylicacid, Behenicacid, Tricosylicacid, Lignocericacid, Pentacosylicacid, Ceroticacid, Heptacosylicacid, Montanicacid, Nonacosylicacid, Melissicacid, Hentriacontylicacid, Lacceroicacid, Psyllicacid, Geddicacid, Ceroplasticacid, Hexatriacontylicacid, Heptatriacontanoicacid, Octatriacontanoicacid, a-Linolenicacid, Stearidonicacid, Eicosapentaenoicacid, Docosahexaenoicacid, Linoleicacid, γ-Linolenicacid, Dihomo-γ-linolenicacid, Arachidonicacid, Adrenicacid, Palmitoleicacid, Vaccenicacid, Paullinicacid, and Oleicacid.

Some non-limiting examples of polyglyceryl esters are Polyglyceryl-4 Caprate, Polyglyceryl-2 Caprate, Polyglyceryl-4 Caprylate, Polyglyceryl-6 Caprylate, Polyglyceryl-6 Caprate, Polyglyceryl-4 Caprylate/Caprate, Polyglyceryl-6 Caprylate/Caprate, Polyglyceryl-3 Cocoate, Polyglyceryl-4 Cocoate, Polyglyceryl-10 Decalinoleate, Polyglyceryl-10 Decaoleate, Polyglyceryl-10 Decacasterate, Polyglyceryl-3 Dicaprate, Polyglyceryl-3 Dicocoate, Polyglyceryl-10 Didecanoate, Polyglyceryl-2 Diisostearate, Polyglyceryl-3 Diisostearate, Polyglyceryl-10 Diisostearate, Polyglyceryl-4 Dilaurate, Polyglycerin-2 Dioleate, Polyglyceryl-3 Dioleate, Polyglyceryl-6 Dioleate, Polyglyceryl-10 Dioleate, Polyglyceryl-6 Dipalmitate, Polyglyceryl-10 Dipalmitate, Polyglyceryl-2 Dipolyhydroxystearate, Polyglyceryl-2 Distearate, Polyglyceryl-3 Distearate, Polyglyceryl-6 Distearate, Polyglyceryl-10 Distearate, Polyglyceryl-10 Heptaoleate, Polyglyceryl-10 Heptastearate, Polyglyceryl-6 Hexaoleate, Polyglyceryl-10 Hexaoleate, Polyglyceryl-2 Isopalmitate, Polyglyceryl-2 Isostearate, Polyglyceryl-4 Isostearate, Polyglyceryl-5 Isostearate, Polyglyceryl-6 Isostearate, Polyglyceryl-10 Isostearate, Polyglyceryl-2 Laurate, Polyglyceryl-3 Laurate, Polyglyceryl-4 Laurate, Polyglyceryl-4 Laurate/Sebacate, Polyglyceryl-4 Laurate/Succinate, Polyglyceryl-5 Laurate, Polyglyceryl-6 Laurate, Polyglyceryl-10 Laurate, Polyglyceryl-3 Myristate, Polyglyceryl-10 Myristate, Polyglyceryl-2 Oleate, Polyglyceryl-3 Oleate, Polyglyceryl-4 Oleate, Polyglyceryl-5 Oleate, Polyglyceryl-6 Oleate, Polyglyceryl-8 Oleate, Polyglyceryl-10 Oleate, Polyglyceryl-3 Palmitate, Polyglyceryl-6 Palmitate, Polyglyceryl-10 Pentalaurate, Polyglyceryl-10 Pentalinoleate, Polyglyceryl-4 Pentaoleate, Polyglyceryl-10 Pentaoleate, Polyglyceryl-3 Pentaricinoleate, Polyglyceryl-6 Pentaricinoleate, Polyglyceryl-10 Pentaricinoleate, Polyglyceryl-4 Pentastearate, Polyglyceryl-6 Pentastearate, Polyglyceryl-10 Pentastearate, Polyglyceryl-3 Polyrisinoleate, Polyglyceryl-6 Polyricinoleate, Polyglyceryl-3 Ricinoleate, Polyglyceryl-2 Sesquiisostearate, Polyglyceryl-2 Sesquioleate, Polyglyceryl-2 Sesquistearate, Polyglyceryl-3 Stearate, Polyglyceryl-2 Stearate, Polyglyceryl-4 Stearate, Polyglyceryl-8 Stearate, Polyglyceryl-10 Stearate, Polyglyceryl-2 Tetraisostearate, Polyglyceryl-6 Tetraoleate, Polyglyceryl-10 Tetraoleate, Polyglyceryl-2 Tetrastearate, Polyglyceryl-2 Triisostearate, Polyglyceryl-3 Triisostearate, Polyglyceryl-10 Trioleate, Polyglyceryl-4 Tristearate, Polyglyceryl Tristearate, Polyglyceryl-10 Tristearate.

Similarly, the polyglycol esters of fatty acids, for example C₃ to C₂₂ fatty acids that can be employed as surfactant include, but are not limited to, Propionicacid, Butyricacid, Valericacid, Caproicacid, Enanthicacid, Caprylicacid, Pelargonicacid, Capricacid, Undecylicacid, Lauricacid, Tridecylicacid, Myristicacid, Pentadecanoicacid, Palmiticacid, Margaricacid, Stearicacid, Nonadecylicacid, Arachidicacid, Heneicosylicacid, Behenicacid, Tricosylicacid, Lignocericacid, Pentacosylicacid, Ceroticacid, Heptacosylicacid, Montanicacid, Nonacosylicacid, Melissicacid, Hentriacontylicacid, Lacceroicacid, Psyllicacid, Geddicacid, Ceroplasticacid, Hexatriacontylicacid, Heptatriacontanoicacid, Octatriacontanoicacid, α-Linolenicacid, Stearidonicacid, Eicosapentaenoicacid, Docosahexaenoicacid, Linoleicacid, γ-Linolenicacid, Dihomo-γ-linolenicacid, Arachidonicacid, Adrenicacid, Palmitoleicacid, Vaccenicacid, Paullinicacid, Oleicacid

Further, non-phospholipid amphiphilic ingredients are also considered for forming vesicles, and such ingredients are typically amphiphilic lipids that hydrate to form layers upon introduction of water or polar solvents such as alcohol, then self-close to form a blister or sac. Such amphiphilic lipids may include alkoxylated fatty carboxylic acid mono-, di-, or triesters; alkoxylated glycerolated fatty mono-, di-, or triesters, sulfonated fatty acid mono-, di-, or triesters, and so on. Examples of alkoxylated fatty esters include those having from about 2 to 500 alkoxy, or ethoxy groups. Examples include PEG (polyethylene glycol) having repeating ethylene oxide units ranging from 2 to 500. The fatty acid esters may be mono-, di-, or triesters, and if di-, or triesters, reacted with alkoxylated and glycerolated moieties. In one preferred embodiment, the alkoxylated fatty acid esters or alkoxylated glycerolated fatty acid esters wherein the fatty acid is an aliphatic carbon chain ranging from about 4 to 30 carbon atoms. Examples of such fatty acid esters include, but are not limited to, monoesters of PEG and fatty carboxylic acids, diesters of PEG and fatty carboxylic acids, or triesters of PEG and fatty carboxylic acids; diesters of PEG, glycerin, and fatty carboxylic acids; triesters of PEG, glycerin, and fatty carboxylic acids. Examples of such molecules include PEG butyrate, PEG isobutyrate, PEG pentanoate, PEG hexanoate, PEG dihexanoate, PEG heptanoate, PEG diheptanoate, PEG octanoate, PEG dioctanoate, PEG nonanoate, PEG dinonanoate, PEG decanoate, PEG dodecanoate, PEG stearate, PEG distearate, PEG isostearate, PEG diisostearate, PEG laurate, PEG dilaurate, PEG myristate, PEG dimyristate, PEG behenate, PEG oleate, PEG dioleate, PEG linoleate, PEG dilinoleate, and so on. Also suitable are esters of glycerin, PEG, and fatty carboxylic acids, such as PEG glycerol dibutyrate, PEG glycerol dipentanoate, PEG glycerol dihexanoate, PEG glyceryl diheptanoate, PEG glycerol dioctanoate, PEG glycerol dinonanoate, PEG glyceryl didecanoate, PEG glyceryl distearate, PEG glyceryl diisostearate, PEG glycerol dilaurate, PEG glycerol dimyristate, PEG glyceryl dibehenate, PEG glyceryl dioleate, PEG glycerol dilinoleate, and so one. In the examples mentioned above, the number of repeating ethylene oxide moieties may range from 1 to 500 (e.g PEG1-500) and, if desired, the number of glycerol moieties may range from 1 to 500, but the molecule should contain enough ethylene oxide and/or glycerol moieties to confer the necessary hydrophilic character to at least a portion of the molecule.

In one embodiment, the sorbitan derivatives are considered as suitable agents for forming non-phospholipid vesicles. Suitable sorbitan derivatives include esters or ethers of sorbitan, which is a heterocyclic ether formed by the dehydration of sorbitol. Sorbitan may be derivatized by ethoxylation and/or esterification of the hydroxyl groups. Suitable acids used for esterification include fatty carboxylic acids having from about 4 to 30 carbon atoms, more preferably, fatty carboxylic acids having 6-22 carbon atoms. Examples of suitable sorbitan derivatives that may be used to form vesicles include PEG derivatives of sorbitan wherein the number of repeating ethylene oxide units ranges from 2 to 200, such as PEG sorbitan beeswax, PEG sorbitan lanolate, PEG sorbitan laurate, PEG sorbitan oleate, PEG sorbitan palmitate, PEG sorbitan perisostearate, PEG sorbitan peroleate, PEG sorbitan stearate, PEG sorbitan tetraoleate, glyceryl/sorbitol/oleate/hydroxystearate, PEG sorbitan cocoate, PEG sorbitan diisostearate, PEG sorbitan isostearate, PEG sorbitan tetrastearate, PEG sorbitan triisostearate; also suitable are polysorbates, which are polymers from sorbitan. For example, Polysorbates 20 to 85 or Polysorbate 20 to 85 acetate are suitable, with the numbers 20 to 85 meaning the number of repeating sorbitan moieties. Sorbitan esters such as such as sorbitan caprylate, cocoate, diisostearate, dioleate, distearate, isostearate, laurate, oleate, olivate, palmitate, sesquiisostearate, sesquioleate, sesquistearate, stearate, triisostearate, trioleate and the like, may also be used to form vesicles.

According to one important embodiment of the present application, octane-1,2-diol or capryl glycol is employed as a co-surfactant for preparing the multilamellar composition of the present application. The amount of octanediol is from about 10 *wt.* % to about 20 *wt.* %. Other ranges would include about 10 *wt*. % to about 12 *wt.* %, about 12 *wt.* % to about 14 *wt.* %, about 14 *wt.* % to about 16 *wt.* %, about 16 *wt.* % to about 18 *wt.* %, or about 18 *wt*. % to about 20 *wt.* %

Suitable solubility enhancer used in the present application is selected from alkanediol or glycol based compounds. An alkanediol based compounds that can be employed in the present application contains at least two carbon atoms, and wherein, any two hydrogen atoms of a saturated aliphatic hydrocarbon of the alkanediol compound are substituted with hydroxyl groups, and wherein, the presence hydroxy groups, (-OH) of the alkanediol may be primary, secondary, or tertiary. In one embodiment, it is disclosed that the presence of two hydroxyl functional groups can be adjacent to each other (vicinal), or can be present in their terminal position as -OH groups or randomly present in any two carbon atom of the carbon chain of C₂₋₂₀. The structure I represents the desired alkanediols of the present application, and wherein, R₁, R₂, R₃, R₄ are independently hydrogen, C₁₋₂₀ alkyl/cycloalkyl, C₁₋₂₀ substituted alkyl/cycloalkyl, hydrocarbyl functional groups. Further, "A" can be a direct bond, independently hydrogen, C₁₋₂₀ alkyl/cycloalkyl, C₁₋₂₀ substituted alkyl/cycloalkyl, and hydrocarbyl functional groups. The term "hydrocarbyl" refers to substituted or unsubstituted alkyl, alkenyl, cycloalkyl, cycloalkenyl or aralkyl, mono-, di- or poly-functional radical that may further contain one or more hetero atoms. However the preferred alkane diol for the present application is 1,3-propane diol, wherein the amount of propane diol is in the range of from about 10 *wt.* % to about 20 *wt.* %. Other ranges would include about 10 *wt*. % to about 12 *wt.* %, about 12 *wt.* % to about 14 *wt.* %, about 14 *wt.* % to about 16 *wt.* %, about 16 *wt.* % to about 18 *wt*. %, or about 18 *wt*. % to about 20 *wt.* %.

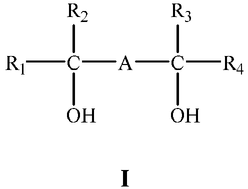

Water is employed to prepare the aqueous multilamellar composition of the present application. The water can be deionized water, double or triple distilled water, reverse osmosis water, or any pure water which is readily available in the market. The quantity required to prepare the multilamellar composition ranges from about 5.0 *wt.* % to about 80 *wt.* %.

In a suitable embodiment, it is contemplated to employ a multilamellar composition of the present application in combination with at least one preservative compound selected from the group consisting of triclosan, 2-methyl-4-isothiazolin-3-one (MIT), 1,2-benzisothiazolin-3-one (BIT), 5-chloro-2-methyl-4-isothiazolin-3-one (CMIT), 2-octyl-4-isothiazolin-3-one (OIT), 3-iodo-2-propynylbutyl-carbamate (IPBC), 3-iodopropynyl-N-phenyl carbamate (IPPC), zinc pyrithione (ZnPy), bronopol, quaternary ammonium compounds, parabens, alkyl parabens, chlorophenisin, benzyl alcohol, organic acids, sorbic acid and their salts, benzoic acid and their salts, salicylic and their salts, potassium sorbate, sodium benzoate, phenoxyethanol, diazolidinyl urea, imidazolidinyl urea, sodium hydroxymethyl glycinate, hydantoins, sodium pyrithione, phenyl ethanol, phenyl propanol, benzalkonium quaternary ammonium compounds, fatty acids and their salts, α-hydroxy acids and their salts, beta acids and their salts, glycerols, hexyl glycerine, tropolones, sisquiterpenes, natural preservatives, and/or ethyl hexyl glycerine

The multilamellar composition of the present application comprising phenylethanol and phenylpropanol is capable of inhibiting or killing yeasts, mold spores, *gram* (+), *gram* (-) bacterial strains, acne causing strains, or odor causing strains, and wherein said microbial strains include, but are not limited to, *Candida tropicalis, Candida albicans, Hansenula anomala, Saccharomyces cerevisiae, Torulaspora delbreuckii, Zygosaccharomyces bailii, Zygosaccharomyces rouxii, Bacillus subtilis, Bacillus cereus, Staphylococcus aureus, Staphylococus epidermidis, Escherichia coli, Salmonella typhimurium, Salmonella enteritidis, Pseudomonas aeruginosa, Aspergillus niger, Aspergillus flavus, Penicillium islandicum, Penicillium citrinum, Penicillium chrysogenum, Fusarium oxysporum, Fusarium graminearum, Fusarium solani, Alternaria alternata, Aspergillus brasiliensis, Burkhodelia cepacia, Enterobacter aerogenes, Enterobacter cloacae, Enterobacter gergoviae, Klebsiella pneumoniae, Proteus vulgaris, Pseudomonas fluorescens, Pseudomonas putida, Penicillium pinophilum Micrococcus luteus, Corynebacterium minutissimum, Corynebacterium xerosis, Corynebacterium jeikeium, Propionibacterium acnes and*/*or Mucor racemosus.*

The aqueous multilamellar composition is stable on storage for at least 2 years at room temperature. The composition is stable for at least 5 freeze/thaw cycles when the temperature is cycled from 50°C to -24°C in every 24 hours or stable for at least 4 weeks at about 45°C.

According to another embodiment of the present application, the aqueous multilamellar composition can be employed in aqueous and non-aqueous based end-user applications comprising cosmetic products, toiletry products, personal care products, oral care products, skin care products, hair care products, household & cleaning products, soap and bath products, industrial and institutional cleaning products, disinfecting products, wound care, sanitary products, agricultural compositions, textile industries, coating industries and/or laundry products. The amount of composition employed in aqueous and non-aqueous based end-user products/compositions is generally in the range of from about 0.01 *wt.* % to about 5.0 *wt.* % of the total composition.

In a further embodiment, the present application provides a process for preparing the above-described aqueous multilamellar composition, wherein said process comprises mixing: (i) about 50 *wt.* % to about 80 *wt.* % of phenylethylalcohol and/or phenylpropylalcohol; (ii) a mixture of (a) about 10 *wt.* % to about 20 *wt.* % of polyglyceryl-4 laurate/sebacate and (b) about 10 *wt.* % to about 20 *wt.* % of polyglyceryl-6 caprylate/caprate; (iii) about 10 *wt.* % to about 20 *wt.* % of octane-1,2-diol; (iv) optionally about 10 *wt.* % to about 20 *wt.* % of 1,3-propanediol; and (v) about 5 *wt.* % to about 80 *wt.* % of water.

A personal care, house hold, cleaning, and/or Industrial & Institutional product comprising an aqueous multilamellar composition of present application required to employ an effective amount of the composition ranging from about 0.01 *wt.* % to 5.0 *wt.* %, relative to the total mass of said product.

One embodiment of the present application relates to a method of killing bacteria, fungi, molds, yeasts and viruses or inhibiting their growth in a cosmetic, personal care, house hold, cleaning, and/or Industrial & Institutional products susceptible to growth of microorganisms.

Yet another embodiment of the present application provides a method for controlled or periodic delivery of a hydrophobic substance of the present application incorporated into a cosmetic or personal care or house hold, cleaning, Industrial & Institutional product.

Further, certain aspects of the present invention are illustrated in detail by way of the following examples. The examples are given herein for illustration of the invention and are not intended to be limiting thereof.

### Example 1:

| **Ingredients** | ***wt. %*** |
|---|---|
| Phenylethylalcohol | 60% |
| Octanediol | 20% |
| Polyglyceryl-4 laurate/sebacate | 20% |
| Polyglyceryl-6 caprylate/caprate | |

### Example 2:

| **Ingredients** | ***wt. %*** |
|---|---|
| Phenylethylalcohol | 60% |
| Octanediol | 20% |
| Propanediol | 10% |
| Polyglyceryl-4 laurate/sebacate | 10% |
| Polyglyceryl-6 caprylate/caprate | |

### Example 3:

| **Ingredients** | ***wt. %*** |
|---|---|
| Phenylethyl alcohol | 60-80 |
| Octan1, 2diol | 10-20 |
| Isotridecylethoxlate | 10-20 |

### Example 4:

| **Ingredients** | ***wt. %*** |
|---|---|
| Phenylethyl alcohol | 60-80 |
| Octanediol | 10-20 |
| Polyglyceryl-4 laurate/sebacate | |
| Polyglyceryl-6 caprylate/caprate | 10-20 |

### Example 5:

| **Ingredients** | ***wt. %*** |
|---|---|
| Phenylethyl propanol | 60-80 |
| Octanediol | 10-20 |
| Polyglyceryl-4 laurate/sebacate | |
| Polyglyceryl-6 caprylate/caprate | 10-20 |

### Example 6:

| **Ingredients** | **%** |
|---|---|
| Phenyethyl alcohol | 55-75 |
| Ocatanediol | 10-20 |
| 1,3 propanediol | 5-10 |
| Polyglyceryl-4 laurate/sebacate | 10-15 |
| Polyglyceryl-6 caprylate/caprate | |

The above described compositions of examples 1 to 6 show clear to light yellow solutions are stable at 45°C for 1 month and pass 5 freeze/thaw cycles.

### Example 7:

Efficacy data of aqueous multilamellar composition demonstrated that it can kill the bacterial strains of *gram* (+) and *gram* (-), yeast and fungi.

A standard screening emulsion composition as described below comprises the composition of Example 1 and 2 in phase C at 1% by *wt*. or no preservative to serve as a control.

| **Phase** | **Ingredients** | ***wt. %*** |
|---|---|---|
| Phase A | Stearic acid, NF | 5.0 |
| | Mineral Oil | 2.5 |
| | Cetyl Alcohol, NF | 1.0 |
| | Ceteareth-5 | 0.5 |
| | PEG 100 Stearate | 1.5 |
| Phase B | DI water | 86.9 |
| | Triethanolamine 99% | 1.0 |
| Phase C | Preservative | 1.0 |
| Phase D | Citric Acid 30% aq. | 0.6 |
| Total | | 100 |

The standard emulsions containing compositions of Example 1 or 2 or 3 at 1% or no preservative (control) were then subjected for a challenge test with various microorganisms following a 28 day double inoculation tests, where the samples were inoculated with either *Gram* (+) bacteria (*Staphylococcus aureus* 6538), a composite of *Gram* (-) bacteria (*Escherichia coli* 8739, *Pseudomonas aeruginosa* 9027 and *Burkholderia cepacia* 25416), whereas the bacteria is inoculated at day 0 and day 21 to a final concentration of about 10^6-7 *cfu*/*ml* and the fungal composite is inoculated at day 0 and day 21 to a final concentration of about 10^5-6 *spores*/*ml.* The inoculated samples are plated at days 2, 7, 14, 21 and 28. The recovery media is Letheen Agar for bacteria and Potato Dextrose agar for fungi. The microorganisms recovered at each time interval are shown in the following Table 1:

**Table 1: PET challenge tests of standard screening emulsion formulation**

| **Treatment** | **Microbial strain** | **day 2** | **day 7** | **day 14** | **day 21** | **day 28** |
|---|---|---|---|---|---|---|
| Control Unpreserved | *Gram* (+) bacteria | 1.2E6 | >1E4 | 6.3E2 | < 10 | >1E4 |
| | *Gram* (-) bacteria | >1E6 | >1E4 | >1E4 | >1E4 | >1E4 |
| | Yeast and mold | 1.3E5 | >1E4 | >1E4 | >1E4 | >1E4 |
| Formulation 1 (1% by wt.) | *Gram* (+) bacteria | <10 | <10 | <10 | <10 | <10 |
| | *Gram* (-) bacteria | <10 | <10 | <10 | <10 | <10 |
| | Yeast and mold | 1.8E4 | <10 | <10 | <10 | <10 |
| Formulation 2 (1% by wt.) | *Gram* (+) bacteria | <10 | <10 | <10 | <10 | <10 |
| | *Gram* (-) bacteria | <10 | <10 | <10 | <10 | <10 |
| | Yeast and mold | 6.1E3 | <10 | <10 | <10 | <10 |
| Formulation 3 (1% by wt.) - non lamellar | *Gram* (+) bacteria | <10 | <10 | <10 | <10 | <10 |
| | *Gram* (-) bacteria | < 10 | < 10 | < 10 | < 10 | <10 |
| | Yeast and mold | 2.8 E 4 | 4.0 E 2 | 1.0 E 1 | <10 | <10 |

As shown in the Table 1, the standard screening emulsion containing either compositions of Example 1 or 2 or 3 at 1% by wt., have significantly reduced the levels of inoculated microorganisms and prevented their growth.

## Claims

1. An aqueous multilamellar composition for delivering a hydrophobic substance comprising:
i. 50 *wt.* % to 80 *wt.* % of phenylethylalcohol and/or phenylpropylalcohol;
ii. a mixture of (a) 10 *wt.* % to 20 *wt*. % of polyglyceryl-4 laurate/sebacate and (b) 10 *wt*. % to 20 *wt*. % of polyglyceryl-6 caprylate/caprate;
iii. 10 *wt*. % to 20 *wt*. % of octane-1,2-diol;
iv. optionally 10 *wt.* % to 20 *wt.* % of 1,3-propanediol; and
v. 5.0 *wt*. % to 80 *wt*. % of water.

2. The aqueous multilamellar composition according to claim 1, wherein said phenylethylalchol and phenylpropylalcohol is a fragrance substance and/or preservative/ antimicrobial activity enhancing substance.

3. The aqueous multilamellar composition according to claim 1, wherein said multilamellar structure is capable of delivering hydrophobic substance in a controlled or periodic release manner.

4. The aqueous multilamellar composition according to claim 1, wherein said mixture of non-ionic surfactants is selected from the group consisting of (a) at least one surfactant having HLB value of 8 to 12 and (b) at least one surfactant having a HLB value of 14 to 17, and wherein, the ratio of mixture of (a) and (b) is from 1:10 to 10:1.

5. The aqueous multilamellar composition according to claim 1, wherein said multilamellar structure is capable of increasing the water solubility of hydrophobic substances by increasing their partitioning towards oil/water interface with largest portion into the water phase.

6. The aqueous multilamellar composition according to claim 1, wherein said multilamellar structure is capable of decreasing vapor pressure of hydrophobic substance and thereby controlled release of pungent odor of phenylethylalcohol and phenylpropylalcohol.

7. The aqueous multilamellar composition according to claim 1, wherein said composition is combined with at least one preservative compounds selected from the group consisting oftriclosan, 2-methyl-4-isothiazolin-3-one (MIT), 1,2-benzisothiazolin-3-one (BIT), 5-chloro-2-methyl-4-isothiazolin-3-one (CMIT), 2-octyl-4-isothiazolin-3-one (OIT), 3-iodo-2-propynylbutyl-carbamate (IPBC), 3-iodopropynyl-N-phenyl carbamate (IPPC), zinc pyrithione (ZnPy), bronopol, quaternary ammonium compounds, parabens, alkyl parabens, chlorophenisin, benzyl alcohol, organic acids, sorbic acid and their salts, benzoic acid and their salts, salicylic and their salts, potassium sorbate, sodium benzoate, phenoxyethanol, diazolidinyl urea, imidazolidinyl urea, sodium hydroxymethyl glycinate, hydantoins, sodium pyrithione, phenyl ethanol, phenyl propanol, benzalkonium quaternary ammonium compounds, fatty acids and their salts, α-hydroxy acids and their salts, beta acids and their salts, glycerols, hexyl glycerine, tropolones, sisquiterpenes, natural preservatives, and/or ethyl hexyl glycerine.

8. The aqueous multilamellar composition according to claim 1, wherein said composition is capable of inhibiting or killing yeasts, mold spores, *gram* (+), *gram* (-) bacterial strains, acne causing strains, or odor causing strains
or
wherein said composition is capable of inhibiting or killing *Candida tropicalis, Candida albicans, Hansenula anomala, Saccharomyces cerevisiae, Torulaspora delbreuckii, Zygosaccharomyces bailii, Zygosaccharomyces rouxii, Bacillus subtilis, Bacillus cereus, Staphylococcus aureus, Staphylococus epidermidis, Escherichia coli, Salmonella typhimurium, Salmonella enteritidis, Pseudomonas aeruginosa, Aspergillus niger, Aspergillus flavus, Penicillium islandicum, Penicillium citrinum, Penicillium chrysogenum, Fusarium oxysporum, Fusarium graminearum, Fusarium solani, Alternaria alternata, Aspergillus brasiliensis, Burkhodelia cepacia, Enterobacter aerogenes, Enterobacter cloacae, Enterobacter gergoviae, Klebsiella pneumoniae, Proteus vulgaris, Pseudomonas fluorescens, Pseudomonas putida, Penicillium pinophilum Micrococcus luteus, Corynebacterium minutissimum, Corynebacterium xerosis, Corynebacterium jeikeium, Propionibacterium acnes and*/*or Mucor racemosus.*

9. The aqueous multilamellar composition according to claim 1, wherein said composition is stable on storage for at least 2 years at room temperature or
wherein the composition is stable for at least 5 freeze/thaw cycles when the temperature is cycled from 50°C to -24°C in every 24 hours or stable for at least 4 weeks at 45°C.

10. The aqueous multilamellar composition according to claim 1, wherein said composition is employed in aqueous and non-aqueous based end-user applications comprising cosmetic products, toiletry products, personal care products, oral care products, skin care products, hair care products, household & cleaning products, soap and bath products, industrial and institutional cleaning products, disinfecting products, wound care, sanitary products, agricultural compositions, textile industries, coating industries and/or laundry products.

11. The aqueous multilamellar composition according to claim 1, wherein the amount of composition employed in aqueous and non-aqueous based end-user products/compositions is in the range of from 0.01 *wt.* % to 5.0 *wt.* % of the total composition.

12. A process for preparing the aqueous multilamellar composition of claim 1, wherein said process comprises mixing:
i. 50 *wt.* % to 80 *wt.* % of phenylethylalcohol and/or phenylpropylalcohol;
ii. a mixture of (a) 10 *wt*. % to 20 *wt*. % of polyglyceryl-4 laurate/sebacate and (b) 10 *wt*. % to 20 *wt*. % of polyglyceryl-6 caprylate/caprate;
iii. 10 *wt*. % to 20 *wt*. % of octane-1,2-diol;
iv. optionally 10 *wt.* % to 20 *wt.* % of 1,3-propanediol; and
v. 5 *wt*. % to 80 *wt*. % of water.

13. A personal care, house hold, and/or cleaning product comprising the aqueous multilamellar composition of claim 1 in an amount ranging from 0.01 *wt.* % to 5.0 *wt.* %, relative to the total mass of said product.

14. A method of killing bacteria, fungi, molds, yeasts and viruses or inhibiting their growth in a cosmetic, personal care, house hold, and/or cleaning products those are susceptible to growth of microorganisms comprising incorporating 0.01 *wt.* % to 5.0 *wt*. % of the preservative composition of claim 1.

15. A method for controlled or periodic delivery of a hydrophobic substance to a cosmetic or personal care or house hold, and/or cleaning products comprising incorporating into said product 0.01 *wt*. % to 5.0 *wt*. % of the preservative composition of claim 1.

## Patentansprüche

1. Eine wässrige multilamellare Zusammensetzung zur Abgabe einer hydrophoben Substanz umfassend:
i. 50 Gew.-% bis 80 Gew.-% Phenylethylalkohol und/oder Phenylpropylalkohol;
ii. eine Mischung aus (a) 10 Gew.-% bis 20 Gew.-% Polyglyceryl-4 Laurat/Sebacat und (b) 10 Gew.-% bis 20 Gew.-% Polyglyceryl-6 Caprylat/Caprat;
iii. 10 Gew.-% bis 20 Gew.-% Octan-1,2-diol;
iv. optional 10 Gew.-% bis 20 Gew.-% 1,3-Propandiol; und
v. 5,0 Gew.-% bis 80 Gew.-% Wasser.

2. Die wässrige multilamellare Zusammensetzung gemäß Anspruch 1, wobei der Phenylethylalkohol und der Phenylpropylalkohol ein Riechstoff und/oder ein Konservierungsmittel/eine antimikrobiell aktivitätssteigernde Substanz ist.

3. Die wässrige multilamellare Zusammensetzung gemäß Anspruch 1, wobei die multilamellare Struktur in der Lage ist, eine hydrophobe Substanz in einer kontrollierten oder periodischen Art und Weise abzugeben.

4. Die wässrige multilamellare Zusammensetzung gemäß Anspruch 1, wobei die Mischung aus nicht-ionischen Tensiden ausgewählt wird aus der Gruppe bestehend aus (a) mindestens einem Tensid mit einem HLB-Wert von 8 bis 12 und (b) mindestens einem Tensid mit einem HLB-Wert von 14 bis 17, und wobei das Mischungsverhältnis von (a) zu (b) 1:10 bis 10:1 beträgt.

5. Die wässrige multilamellare Zusammensetzung gemäß Anspruch 1, wobei die multilamellare Struktur in der Lage ist, die Wasserlöslichkeit hydrophober Substanzen zu erhöhen, indem sie ihre Aufteilung in Richtung Öl-/Wasser-Grenzfläche mit dem größten Anteil in die Wasserphase erhöht.

6. Die wässrige multilamellare Zusammensetzung gemäß Anspruch 1, wobei die multilamellare Struktur in der Lage ist, den Dampfdruck der hydrophoben Substanz zu verringern und dadurch eine kontrollierte Freisetzung von stechendem Geruch von Phenylethylalkohol und Phenylpropylalkohol zu bewirken.

7. Die wässrige multilamellare Zusammensetzung gemäß Anspruch 1, wobei die Zusammensetzung mit wenigstens einem Konservierungsmittel ausgewählt aus der Gruppe bestehend aus Triclosan, 2-Methyl-4-isothiazolin-3-on (MIT), 1,2-Benzisothiazolin-3-on (BIT), 5-Chlor-2-methyl-4-isothiazolin-3-on (CMIT), 2-Octyl-4-isothiazolin-3-on (OIT), 3-lod-2-propynylbutylcarbamat (IPBC), 3-lodpropynyl-N-phenylcarbamat (IPPC), Zinkpyrithion (ZnPy), Bronopol, quaternäre Ammoniumverbindungen, Parabene, Alkylparabene, Chlorphenisin, Benzylalkohol, organische Säuren, Sorbinsäure und ihre Salze, Benzoesäure und ihre Salze, Salicyl und ihre Salze, Kaliumsorbat, Natriumbenzoat, Phenoxyethanol, Diazolidinylharnstoff, Imidazolidinylharnstoff, Natriumhydroxymethylglycinat, Hydantoine, Natriumpyrithion, Phenylethanol, Phenylpropanol, Benzalkonium quaternäre Ammoniumverbindungen, Fettsäuren und deren Salze, α-Hydroxysäuren und deren Salze, beta-Säuren und deren Salze, Glycerine, Hexylglycerin, Tropolone, Sesquiterpene, natürliche Konservierungsmittel und/oder Ethylhexylglycerin.

8. Die wässrige multilamellare Zusammensetzung gemäß Anspruch 1, wobei die Zusammensetzung in der Lage ist, Hefen, Schimmelpilzsporen, *gram* (+)-, *gram*(-)-Bakterienstämme, Akne verursachende Stämme oder geruchsverursachende Stämme zu hemmen oder abzutöten
oder
wobei die Zusammensetzung in der Lage ist, *Candida tropicalis, Candida albicans, Hansenula anomala, Saccharomyces cerevisiae, Torulaspora delbreuckii, Zygosaccharomyces bailii, Zygosaccharomyces rouxii, Bacillus subtilis, Bacillus cereus, Staphylococcus aureus, Staphylococus epidermidis, Escherichia coli, Salmonella typhimurium, Salmonella enteritidis, Pseudomonas aeruginosa, Aspergillus niger, Aspergillus flavus, Penicillium islandicum, Penicillium citrinum, Penicillium chrysogenum, Fusarium oxysporum, Fusarium graminearum, Fusarium solani, Alternaria alternata, Aspergillus brasiliensis, Burkhodelia cepacia, Enterobacter aerogenes, Enterobacter cloacae, Enterobacter gergoviae, Klebsiella pneumoniae, Proteus vulgaris, Pseudomonas fluorescens, Pseudomonas putida, Penicillium pinophilum Micrococcus luteus, Corynebacterium minutissimum, Corynebacterium xerosis, Corynebacterium jeikeium, Propionibacterium acnes* und/oder *Mucor racemosus* zu hemmen oder abzutöten.

9. Die wässrige multilamellare Zusammensetzung gemäß Anspruch 1, wobei die Zusammensetzung bei Lagerung für mindestens 2 Jahre bei Raumtemperatur stabil ist oder
wobei die Zusammensetzung für mindestens 5 Gefrier-/Tauzyklen stabil ist, wenn die Temperatur alle 24 Stunden von 50°C bis -24°C verändert wird oder für mindestens 4 Wochen bei 45°C gehalten wird.

10. Die wässrige multilamellare Zusammensetzung gemäß Anspruch 1, wobei die Zusammensetzung in Endverbraucheranwendungen auf wässriger und nicht-wässriger Basis eingesetzt wird, die kosmetische Produkte, Toilettenartikel, Körperpflegeprodukte, Mundpflegeprodukte, Hautpflegeprodukte, Haarpflegeprodukte, Haushalts- und Reinigungsprodukte, Seifen- und Badeprodukte, industrielle und institutionelle Reinigungsprodukte, Desinfektionsprodukte, Wundversorgung, Hygieneprodukte, landwirtschaftliche Zusammensetzungen, Textilindustrie-, Beschichtungsindustrie- und/oder Wäschereiprodukte umfassen.

11. Die wässrige multilamellare Zusammensetzung gemäß Anspruch 1, wobei die Menge der Zusammensetzung, die in wässrigen und nicht-wässrigen Endverbraucherprodukten/Zusammensetzungen verwendet wird, im Bereich von 0,01 Gew.-% bis 5,0 Gew.-% der Gesamtzusammensetzung liegt.

12. Ein Verfahren zur Herstellung der wässrigen multilamellaren Zusammensetzung gemäß Anspruch 1, wobei das Verfahren das Mischen umfasst:
i. 50 Gew.-% bis 80 Gew.-% Phenylethylalkohol und/oder Phenylpropylalkohol;
ii. eine Mischung aus (a) 10 Gew.-% bis 20 Gew.-% Polyglyceryl-4 Laurat/Sebacat und (b) 10 Gew.-% bis 20 Gew.-% Polyglyceryl-6 Caprylat/Caprat;
iii. 10 Gew.-% bis 20 Gew.-% Octan-1,2-diol;
iv. optional 10 Gew.-% bis 20 Gew.-% 1,3-Propandiol; und
vi. 5,0 Gew.-% bis 80 Gew.-% Wasser.

13. Ein Körperpflege-, Haushalts- und/oder Reinigungsprodukt umfassend die wässrige multilamellare Zusammensetzung gemäß Anspruch 1 in einer Menge im Bereich von 0,01 Gew.-% bis 5,0 Gew.-% bezogen auf die Gesamtmenge des Produkts.

14. Ein Verfahren zum Abtöten von Bakterien, Pilzen, Schimmelpilzen, Hefen und Viren oder zum Hemmen ihres Wachstums in einem Kosmetik-, Körperpflege-, Haushalts- und/oder Reinigungsprodukt, das für das Wachstum von Mikroorganismen anfällig ist, umfassend das Einbringen von 0,01 Gew.-% bis 5,0 Gew.-% der konservierenden Zusammensetzung gemäß Anspruch 1.

15. Ein Verfahren zur kontrollierten oder periodischen Abgabe einer hydrophoben Substanz an ein Kosmetik- oder ein Körperpflege- oder ein Haushalts- und/oder ein Reinigungsprodukt umfassend das Einbringen von 0,01 Gew.-% bis 5,0 Gew.-% der konservierenden Zusammensetzung gemäß Anspruch 1 in das Produkt.

## Revendications

1. Composition aqueuse multilamellaire pour l'administration d'une substance hydrophobe comprenant :
i. 50 % en poids à 80 % en poids d'alcool phényléthylique et/ou d'alcool phénylpropylique ;
ii. un mélange de (a) 10 % en poids à 20 % en poids de laurate/sébacate de polyglycéryl-4 et de (b) 10 % en poids à 20 % en poids de caprylate/caprate de polyglycéryl-6 ;
iii. 10 % en poids à 20 % en poids d'octane-1,2-diol ;
iv. éventuellement 10 % en poids à 20 % en poids de 1,3-propanediol ; et
v. 5,0 % en poids à 80 % en poids d'eau.

2. Composition aqueuse multilamellaire selon la revendication 1, ledit alcool phényléthylique et alcool phénylpropylique étant une substance de fragrance et/ou une substance amplifiant l'activité conservatrice/antimicrobienne.

3. Composition aqueuse multilamellaire selon la revendication 1, ladite structure multilamellaire étant capable d'administrer une substance hydrophobe d'une manière de libération contrôlée ou périodique.

4. Composition aqueuse multilamellaire selon la revendication 1, ledit mélange de tensioactifs non ioniques étant choisi dans le groupe constitué par (a) au moins un tensioactif possédant une valeur de HLB (hydrophylic-lipophilic balance - équilibre hydrophile-lipophile) de 8 à 12 et (b) au moins un tensioactif possédant une valeur de HLB de 14 à 17, et le rapport de mélange de (a) et (b) étant de 1:10 à 10:1.

5. Composition aqueuse multilamellaire selon la revendication 1, ladite structure multilamellaire étant capable d'augmenter la solubilité dans l'eau de substances hydrophobes par l'augmentation de leur partitionnement vis-à-vis d'une interface huile/eau, la plus grande partie se trouvant dans la phase aqueuse.

6. Composition aqueuse multilamellaire selon la revendication 1, ladite structure multilamellaire étant capable de diminuer la pression de vapeur d'une substance hydrophobe et ainsi la libération contrôlée de l'odeur piquante de l'alcool phényléthylique et de l'alcool phénylpropylique.

7. Composition aqueuse multilamellaire selon la revendication 1, ladite composition étant combinée avec au moins un composé conservateur choisi dans le groupe constitué par triclosan, 2-méthyl-4-isothiazolin-3-one (MIT), 1,2-benzisothiazolin-3-one (BIT), 5-chloro-2-méthyl-4-isothiazolin-3-one (CMIT), 2-octyl-4-isothiazolin-3-one (OIT), 3-iodo-2-propynylcarbamate de butyle (IPBC), 3-iodopropynylcarbamate de N-phényle (IPPC), zinc pyrithione (ZnPy), bronopol, composés d'ammonium quaternaire, parabènes, alkylparabènes, chlorophenisin, alcool benzylique, acides organiques, acide sorbique et leurs sels, acide benzoïque et leurs sels, salicylique et leurs sels, sorbate de potassium, benzoate de sodium, phénoxyéthanol, diazolidinylurée, imidazolidinylurée, hydroxyméthylglycinate de sodium, hydantoïnes, pyrithione de sodium, phényléthanol, phénylpropanol, composés d'ammonium quaternaire du type benzalkonium, acides gras et leurs sels, α-hydroxyacides et leurs sels, bêtaacides et leurs sels, glycérols, hexylglycérine, tropolones, sesquiterpènes, conservateurs naturels, et/ou éthylhexylglycérine.

8. Composition aqueuse multilamellaire selon la revendication 1, ladite composition étant capable d'inhiber ou de tuer des levures, des spores de moisissures, des souches bactériennes gram (+), gram (-), des souches causant l'acné ou des souches causant une odeur
ou
ladite composition étant capable d'inhiber ou de tuer *Candida tropicalis, Candida albicans, Hansenula anomala, Saccharomyces cerevisiae, Torulaspora delbreuckii, Zygosaccharomyces bailii, Zygosaccharomyces rouxii, Bacillus subtilis, Bacillus cereus, Staphylococcus aureus, Staphylococus epidermidis, Escherichia coli, Salmonella typhimurium, Salmonella enteritidis, Pseudomonas aeruginosa, Aspergillus niger, Aspergillus flavus, Pénicillium islandicum, Pénicillium citrinum, Pénicillium chrysogenum, Fusarium oxysporum, Fusarium graminearum, Fusarium solani, Alternaria alternata, Aspergillus brasiliensis, Burkhodelia cepacia, Enterobacter aerogenes, Enterobacter cloacae, Enterobacter gergoviae, Klebsiella pneumoniae, Proteus vulgaris, Pseudomonas fluorescens, Pseudomonas putida, Pénicillium pinophilum, Micrococcus luteus, Corynebacterium minutissimum, Corynebacterium xerosis, Corynebacterium jeikeium, Propionibacterium acnes* et/ou *Mucor racemosus.*

9. Composition aqueuse multilamellaire selon la revendication 1, ladite composition étant stable lorsqu'elle est stockée pendant au moins 2 ans à température ambiante ou ladite composition étant stable pendant au moins 5 cycles gel/dégel lorsque la température est sous forme de cycle de 50 °C à -24 °C toutes les 24 heures ou stable pendant au moins 4 semaines à 45 °C.

10. Composition aqueuse multilamellaire selon la revendication 1, ladite composition étant utilisée dans des applications d'utilisateur final à base aqueuse et non aqueuse comprenant des produits cosmétiques, des produits de toilette, des produits de soin personnel, des produits d'hygiène buccale, des produits de soin de la peau, des produits de soin capillaire, des produits ménagers et de nettoyage, des savons et des produits de bain, des produits de nettoyages industriels et institutionnels, des produits désinfectants, des soins des plaies, des produits sanitaires, des compositions agricoles, des produits des industries textiles, des industries de revêtement et/ou des produits à lessive.

11. Composition aqueuse multilamellaire selon la revendication 1, la quantité de composition utilisée dans des produits/compositions d'utilisateur final à base aqueuse et non aqueuse se situant dans la plage de 0,01 % en poids à 5,0 % en poids de la composition totale.

12. Procédé pour la préparation de la composition aqueuse multilamellaire selon la revendication 1, ledit procédé comprenant le mélange de :
i. 50 % en poids à 80 % en poids d'alcool phényléthylique et/ou d'alcool phénylpropylique ;
ii. un mélange de (a) 10 % en poids à 20 % en poids de laurate/sébacate de polyglycéryl-4 et de (b) 10 % en poids à 20 % en poids de caprylate/caprate de polyglycéryl-6 ;
iii. 10 % en poids à 20 % en poids d'octane-1,2-diol ;
iv. éventuellement 10 % en poids à 20 % en poids de 1,3-propanediol ; et
v. 5 % en poids à 80 % en poids d'eau.

13. Produit de soin personnel, ménager et/ou de nettoyage comprenant la composition aqueuse multilamellaire selon la revendication 1 en une quantité se situant dans la plage de 0,01 % en poids à 5,0 % en poids, par rapport à la masse totale dudit produit.

14. Procédé pour tuer des bactéries, des champignons, des moisissures, des levures et des virus ou pour inhiber leur croissance dans des produits cosmétiques, de soin personnel, ménagers et/ou de nettoyage ceux-ci étant susceptibles à la croissance de microorganismes comprenant l'incorporation de 0,01 % en poids à 5,0 % en poids de la composition conservatrice selon la revendication 1.

15. Procédé pour la libération contrôlée ou périodique d'une substance hydrophobe à des produits cosmétiques, de soin personnel, ménagers et/ou de nettoyage comprenant l'incorporation dans ledit produit de 0,01 % en poids à 5,0 % en poids de la composition conservatrice selon la revendication 1.
